(19) Europäisches Patentamt
European Patent Office
Office européen des brevets

(11) EP 0 970 998 A1

(12) **EUROPÄISCHE PATENTANMELDUNG**

(43) Veröffentlichungstag:
**12.01.2000 Patentblatt 2000/02**

(51) Int. Cl.[7]: **C08L 91/06**, A61K 47/14

(21) Anmeldenummer: **99112028.8**

(22) Anmeldetag: **22.06.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **07.07.1998 DE 19830240**

(71) Anmelder:
- **Chemtec Leuna Gesellschaft für Chemie und Technologie mbH
06236 Leuna (DE)**
- **Schümann Sasol GmbH & Co. KG
20457 Hamburg (DE)**

(72) Erfinder:
- **Klimek, Irene, Dipl.-Chem.
06122 Halle (DE)**
- **Becker, Karl, Dr.
06237 Leuna (DE)**
- **Kuhlei, Harald
22605 Hamburg (DE)**
- **Meinicke, Steffen
06217 Merseburg (DE)**
- **Liebscher, Gert, Dr.
06231 Bad Dürrenberg (DE)**

(74) Vertreter:
**Schinke, Herbert, Dr. Dr.,
Patentanwaltskanzlei
Postfach 11 11
06234 Leuna (DE)**

(54) **Wachsester mit vaselineähnlicher Konsistenz**

(57) Die Erfindung betrifft Wachsester mit vaselineähnlicher Konsistenz auf Basis pflanzlicher Rohstoffe. Die Eigenschaften können für den jeweiligen Einsatzzweck zugeschnitten werden.

Erfindungsgemäß werden die Wachsester mit vaselineähnlicher Konsistenz aus einwertigen Alkoholen und Dicarbonsäuren entwickelt, die aus mindestens zwei verschiedenen geradkettigen und/oder verzweigten Alkoholen - und Dicarbonsäure gebildet sind.

Das Verhältnis der beiden zur Veresterung eingesetzten Alkoholkomponenten beträgt in der Regel 1 : 1 bis 0,5 : 1.

Die Verwendung der Wachsester erfolgt als Komponente für Cremes und Salben, vorzugsweise in der Pharmazie, Medizin, Dermatologie und Kosmetik.

EP 0 970 998 A1

## Beschreibung

Die Erfindung betrifft Wachsester mit vaselineähnlicher Konsistenz auf Basis pflanzlicher Rohstoffe. Die Eigenschaften können für den jeweiligen Einsatzzweck zugeschnitten werden.

Unter Vaseline versteht man halbfestes Paraffin mit salbenartigem Charakter, fadenziehend, homogen, in dünner Schicht durchscheinend, in Wasser unlöslich.

Die derzeit eingesetzte Vaseline wird aus Destillationsrückständen paraffinbasischer Erdöle oder aus den bei der Entparaffinierung schwerer Erdöldestillate verbleibenden Rückständen (Petrolatum) gewonnen. Chemisch gesehen ist Vaseline ein Gemisch aus Paraffinkohlenwasserstoffen, viskosen Mineralölen und geringen Mengen linearer Paraffine.

Vaseline findet z.B. als Salbengrundlage, Lederfett, Schmiermittel und Ziehfett, sowohl in kosmetisch/pharmazeutischen als auch technischen Bereichen Anwendung Für Vaseline, die vorzugsweise in der pharmazeutischen und kosmetischen Industrie als Salben- bzw. Cremegrundlage Verwendung findet, steht der salbenartige Charakter im Vordergrund, während Eigenschaften wie fadenziehend hier nicht relevant sind, im Hintergrund stehen.

Spezifikationen für Vaseline für kosmetische und pharmazeutische Anwendungen sind z. B. unter der Bezeichnung als Gelbe und Weiße Vaseline im DAB 9,1425ff und DGK (Deutsche Gesellschaft für wissenschaftliche und angewandte Kosmetik e.V.) veröffentlicht. Die im Handel erhältliche Vaseline, auch für die Salben- und Cremes eingesetzte ist also ein Mineralfett.

In jüngster Zeit jedoch sind mehrere Veröffentlichungen erschienen, die gegen eine Verwendung von mineralölstämmigen Produkten, wie Vaseline oder Kohlenwasserstoffgele aus der Erdölfraktionierung, sprechen. Zum einen werden immer wieder Hautreizungen und Unverträglichkeitserscheinungen beobachtet, zum anderen besteht der Verdacht, daß durch diese Substanzen neoplastisches Wachstum auf der Haut ausgelöst werden könnte. Daher wird nach Alternativen für derartige Produkte gesucht.

Die DE 44 17 640 schützt eine Salbengrundlage auf Kohlenwasserstoffbasis, die sich zusammensetzt aus Polyethylen hoher und niederer Dichte, Vinylestern oder -ethern oder kristallinen Paraffinen in niedermolekularen hydrophoben Vinylpolymeren.

Derartige Vinylester oder -ether bzw. niedermolekulare Vinylpolymere haben sich jedoch nicht als Kosmetikrohstoff durchgesetzt, da sie in der Fachwelt in toxikologischer und ökotoxikologischer Hinsicht ebenfalls umstritten sind.

Ziel ist es daher, halbfeste Produkte mit vaselineähnlichen Eigenschaften auf Pflanzenölbasis wie Fettsauren und Fettalkoholen zu gewinnen, die toxikologisch unbedenklich sind, sich in gleichbleibender Qualität herstellen lassen und eine gute Verträglichkeit mit den üblichen Rezepturbestandteilen aufweisen.

Erfindungsgemäß werden Wachsester mit vaselineähnlicher Konsistenz aus einwertigen Alkoholen und Dicarbonsäuren entwickelt, die aus mindestens zwei verschiedenen geradkettigen und/oder verzweigten Alkoholen und Dicarbonsäure gebildet sind.

Das Verhältnis der beiden zur Veresterung eingesetzten Alkoholkomponenten beträgt in der Regel 1 : 1 bis 0.5 : 1, wobei 2 Varianten bevorzugt werden:

a) geradkettige Alkohole unterschiedlicher Kettenlänge (C-Zahl) z.B. C16/C18
b) geradkettiger Alkohol und verzweigter Alkohol z.B. C16 geradkettig/C36 verzweigter Alkohol.

Die erfindungsgemäßen Wachsester sind zu mindestens 80 % durch folgende Struktur gekennzeichnet:

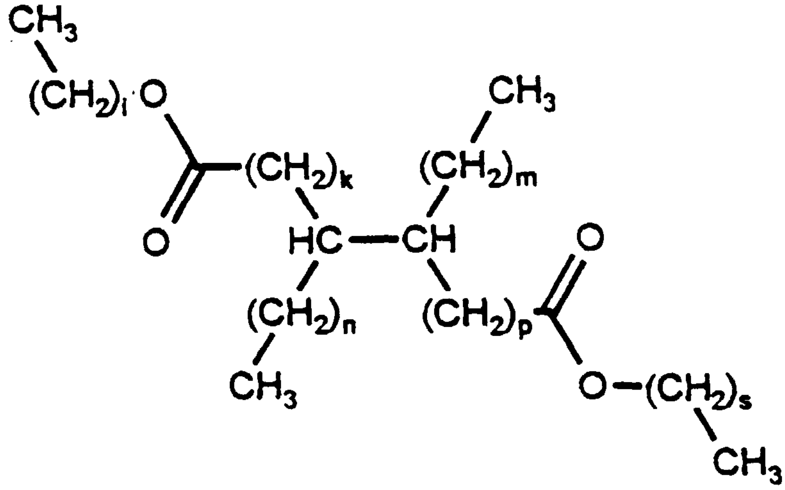

wobei

i einen Zahlenwert von 13 bis 35,
s einen Zahlenwert von 13 bis 35,
die Summe von k+m+n+p etwa 32 beträgt und
$(CH_2)_i$ oder $(CH_2)_s$ geradkettig oder geradkettig und verzweigt sein kann.

Als einwertige geradkettige Alkohole werden zweckmäßig solche mit 14 bis 24 C-Atomen, vorzugsweise mit 16 oder 18 C-Atomen, eingesetzt.

Als einwertige verzweigtkettige Alkohole werden solche mit 14 bis 36 C-Atomen, vorzugsweise Guerbetalkohole, wie 2-Octyldodecanol, oder Isostearylalkohol, eingesetzt.

Bei den Guerbetalkoholen handelt es sich z.B. um 2-Hexyldecan-1-ol, 2-Octyldecan-1-ol oder 2-Octyldodecan-1-ol (letzteres wird unter dem Markennamen Guerbitol von Fa. Henkel gehandelt) Diese Alkohole wurden durch die sog. Guerbet-Reaktion hergestellt.

Die Guerbet-Reaktion ist eine Selbstkondensation von Alkoholen unter Einfluß von Natrium oder Kupfer bei 200 °C und erhöhtem Druck. Man nimmt an, daß unter den Reaktionsbedingungen der Alkohol zunächst zum Aldehyd dehydriert wird, dieser eine Aldol-Addition mit sich selbst eingeht und das Kondensationsprodukt anschliessend zum Alkohol wird.

Vorzugsweise werden als Dicarbonsäure Dimersäuren eingesetzt, die durch Dimerisierung von Gemischen aus ungesättigten Fettsäuren hergestellt werden, die mindestens einen C18:1-Anteil von 80 % aufweisen und durch Spaltung von Pflanzenölen mit einem hohen C18:1-Anteil (Ölsäureanteil) gewonnen wurden.

Die verwendeten handelssüblichen Dimersäuren sind Gemische aus acyclischen Dicarbonsäuren mit durchschnittlich 36 C-Atomen, die in der Regel ≥ 75 % Dimere, etwa 15 bis 20 % Trimere und höchstens 5% Monomere enthalten.

Sie werden durch katalysierte Dimerisierung ungesättigter Fettsäuren mit vorzugsweise 18 C-Atomen gewonnen. Die ungesättigten Fettsäuren wurden durch Spaltung von Pflanzenölen, wie oben beschrieben, hergestellt.

Dabei entstehen zunächst Gemische aus cyclischen und acyclischen Dicarbonsäuren mit durchschnittlich 36 C-Atomen, wobei als Nebenprodukte verzweigte $C_{18}$-Fettsäuren, nicht umgesetzte Fettsäuren und Trimerfettsäuren auftreten können. Durch destillative Aufarbeitung stehen dann die acyclischen Gemische mit etwa 75 % Dimeren, 15 bis 20 % Trimeren und 5 % Monomeren zur Verfügung wobei der Dimeranteil je nach destillativem Aufwand bis zu 95 % betragen kann.

Ein Verfahren zur Herstellung der Wachsester mit vaselineähnlicher Konsistenz besteht darin, Dicarbonsäuren, vorzugsweise Dimersäure, mit zwei verschiedenen geradkettigen und/oder verzweigten einwertigen Alkoholen in Gegenwart eines sauren Katalysators bei Temperaturen von 100 bis 160 °C umzusetzen, wobei sich ggfs. eine alkalische Wäsche anschließt.

Als saure Katalysatoren kommen insbesondere organische Säuren wie Essigsaure, Oxalsäure, Weinsäure oder anorganische Säuren wie Schwefelsäure oder Phosphorsäure in Frage.

Die Veresterungsreaktion findet vorzugsweise in der Schmelze bei Temperaturen von 100 bis 160 °C in einem heiz- und kühlbaren Rührbehälter unter Vakuum von -0,5 bis -1,5 bar oder durch Austreiben des Wassers mittels Inertgasstrom statt. Die Reaktionskontrolle erfolgt wie üblich über die Verfolgung der Säurezahlabnahme mittels Säurezahlbestimmung (DIN 53 402). Nach Erreichen des gewünschten Umsatzgrades kann sich eine alkalische Wäsche zur Produktreinigung bzw. zum Entfernen von Katalysatorspuren anschließen.

Der saure Katalysator wird dabei nach der Veresterung durch Alkali oder Erdalkali neutralisiert (alkalische Wäsche). Verwendet werden können 5 bis 10%ige Natronlauge, Lithiumhydroxid, Calciumhydroxid oder eine 5 bis 30%ige $NaHCO_3$- oder $Na_2CO_3$-Lösung. Vorzugsweise wird eine 5 bis 30%ige $NaHCO_3$- oder $Na_2CO_3$-Lösung zugesetzt.

Die Verwendung der Wachsester erfolgt als Komponente für Cremes und Salben, vorzugsweise in der Pharmazie, Medizin, Dermatologie und Kosmetik.

Die Wachsester können aber auch für technische Anwendungen wie Kabelschmierfett oder als Komponente in Lederpflegemitteln eingesetzt werden.

Die Wachsester sind farb- und geruchlos. Die Eigenschaften, wie z. B. Schmelzbereich oder Viskosität sind in einem weiten Bereich variabel. Das Produkt eignet sich besonders zum Einsatz als Basis für eine Salbengrundlage und zur Emulgierung empfindlicher Wirkstoffe in der Pharmazie und Kosmetik.

Das erfindungsgemäße, nativstämmige Produkt kann in kosmetischen und pharmazeutischen Formulierungen mineralölstämmige Vaseline ersetzen. Gegenüber mineralölstämmiger Vaseline weist das erfindungsgemäße Produkt weiterhin eine verbesserte Emulsionsatabilität und Homogenität auf und seine Polarität und Konsistenz ist gezielt einstellbar.

Ausführungsbeispiele:

Beispiel 1

In einer mit Außenheizung versehenen Laborglasapparatur (Mehrhaiskolben) mit Rührer, Einleitungsrohr für Inertgas, Ableitung für Inertgas und Reaktionswasser und Temperaturmessung werden 200 g Dimersäure, 80 g Stearylalkohol und 110 g Cetylalkohol in das mit Stickstoff beatmete Reaktionsgefäß eingetragen und auf eine Temperatur von 120 ± 5 °C gebracht. Die Rührgeschwindigkeit wird so eingestellt, daß eine gute Durchmischung der Reaktionskomponenten gewährleistet ist. Anschließend wird der Inertgasstrom geregelt und 0,1 ml konzentrierte Schwefelsäure zugesetzt. Der Verlauf der Umsetzung wird über Säurezahlbestimmung geprüft und bis zur Säurezahl von 5 bis 15 mg KOH/g umgesetzt. Hierzu wird in Prüfintervallen eine Probe aus dem Reaktorkolben entnommen.

Das Endprodukt weist folgende allgemeine Wachskenndaten auf:

Tabelle 1

| Säurezahl (DIN 53 402) mg KOH/g | Verseifungszahl (DIN 53 401) mg KOH/g | Viskosität (DIN 51 562) $mm^2/s$ | Erstarrungspunkt (DIN ISO 2207) °C | Penetration (DIN 51 580) 1/10 mm |
|---|---|---|---|---|
| 5,8 | 105 | 20,4 | 38 | 280 |

Nach den Ergebnissen der durchgeführten toxikologischen Untersuchungen ist das Produkt weder toxisch ($LD_{50}$ p.o. Ratte: > 5000 mg/kg KM) noch haut- oder augenreizend, bewirkt weder eine Sensibilisierung der Haut noch ist es mutagen und erfüllt die 155, BGA-Mitteilung.

Beispiele 2 bis 5:

Die Veresterungsreaktion findet in einem heiz- und kühlbaren 70 l-Rührreaktor im schmelzflüssigen Zustand der Reaktanten statt. Das sich bildende Reaktionswasser wird mittels Vakuum abgezogen. Die Tabelle 2 zeigt die Einsatzprodukte, Reaktionsbedingungen und die allgemeinen wachstypischen Kennwerte.

Im Anschluß an die Veresterung kann die Schmelze mit einer 5 bis 10%igen $NaHCO_3$- oder $Na_2CO_3$-Lösung gewaschen werden, um saure Katalysatorspuren zu entfernen. Dazu werden 1 bis 5 l der Lösung bei Temperaturen oberhalb des Schmelzbereiches bis max. 90 °C in die Schmelze dosiert und durch Rühren ca. 10 min innig vermischt. Anschließend läßt man die wäßrige Phase am Boden des Reaktors absetzen und zieht das Waschwasser durch ein Bodenventil ab. Das so gereinigte Produkt kann nochmals mittels Vakuum entwässert werden, wird anschließend gekühlt und in entsprechende Gefäße gefüllt.

Tabelle 2

| Einsatzprodukte | | | | | |
|---|---|---|---|---|---|
| | Dicarbonsäure | Alkohol 1 | Alkohol 2 | Katalysator | Nachbehandlung |
| 2 | 25 kg Dimersäure | 5 kg Stearylalk | 7 kg Cetylalk. | 50 g $H_2SO_4$ | 0,5 l $NaHCO_3$ (10%ig) |
| 3 | 25 kg Dimersäure | 3 kg Isostearylalk. | 10 kg Stearylalk. | 50 g $H_3PO_4$ | 0,8 l $NaHCO_3$ (10%ig) |
| 4 | 25 kg Dimersäure | 5 kg Isostearylalk. | 8 kg Eicosanol | 50 g $H_2SO_4$ | 1,0 l $Na_2CO_3$ (5%ig) |
| 5 | 25 kg Dimersäure | 11 kg Stearylalk. | 5 kg Guerbitol | 50 g $H_3PO_4$ | 1,8 l $Na_2CO_3$ (5%ig) |
| Kenndaten der Produkte | | | | | |
| | Säurezahl (DIN 53402) | Verseifungszahl (DIN 53401) | kinematische Viskosität (DIN 51562) | Erstarrungspunkt (DIN ISO 2207) | Penetraton (DIN 51580) |
| 2 | 7,9 mg KOH/g | 113,0 mg KOH/g | 20,6 $mm^2/s$ | 34,5 °C | 282*[1]/10 mm |
| 3 | 5,2 mg KOH/g | 115,9 mg KOH/g | 22,2 $mm^2/s$ | 35,22 °C | 280*[1]/10 mm |
| 4 | 8,6 mg KOH/g | 118,4 mg KOH/g | 23,1 $mm^2/s$ | 35,2 °C | 286*[1]/10 mm |
| 5 | 9,3 mg KOH/g | 79,3 mg KOH/g | 24,9 $mm^2/s$ | 28,5 °C | > 300*[1]/10 mm |

Zur Charakterisierung wurde eine spezielle HPLC-Methode entwickelt. Es handelt sich um eine isokratische NARP-Variante mit UV-Detektion (NARP = Non Aqueous Reversed Phase).
Bei der Methodenentwicklung wurde neben hoher Selektivität auch auf Detektierbarkeit im UV orientiert. Es wurde festgestellt, daß sich die vorgeschlagene Verbindung im System Aceton/Acetonitril unter Verwendung eines speziellen Umkehrphasenmaterials sehr gut charakterisieren läßt. Wesentlicher Vorteil der Methode ist neben der Selektivität für die Inhaltsstoffe die Detektierbarkeit im UV.
Unter den festgelegten HPLC-Bedingungen gelingt die UV-Detektion bei 210 nm, im Absorptionsminimum von Aceton. Die erhaltenen Peakmuster erlauben den Vergleich von Produktmustern und die Erkennung typischer Peakgruppen mit C16-, C18- oder gemischter Substitution technischer Dimersäure. Aus der Retentionsfolge sind unsubstituzerte Anteile und substituierte Mono-, Di- und Trimere unterscheidbar.
Die Auswertung erfolgt in Flächenprozent. Es können sowohl Einzelpeaks als auch Peakgruppen bewertet werden. Die nachfolgende Tabelle enthalt die chromatographischen Bedingungen:

| | |
|---|---|
| HPLC-System | 1090 M (Hewlett-Packard) mit Diodenarray-Detektor 1040A |
| Trennsäure | RP selectB, 5 µm, 250x4 mm (Merck) |
| Eluent A | Acetonitril/Aceton 6/4 (V/V)+0,1 % Phosphorsäure |
| Fließgeschwindigkeit | 1,0 ml/min |
| Säulenraumtemperatur | 50 Grad |
| Detektion | UV, 210 nm (Bandbreite 10 nm, Ref. 450,80 nm) |
| Probenvorbereitung | ca. 30 mg/10 ml Aceton |
| Injektion | 20 µl |
| Analysenzeit | 20 min |

Beispiel 6

Verwendung der erfindungsgemäßen Wachsester in einer W/O-Creme

| Rezepturbestandteile | Teile % | Funktion |
|---|---|---|
| Wachsester hergestellt gemäß | | |
| Beispiel 1 | 14 | Emollient |
| Hexyl Laurate | 8 | Solvent |
| PEG-1 Glyceryl-Sorbitan Isostearate (and) Paraffin Wax | 8 | Emulgator |
| PEG-22/Dodecyl Glycol Copolymer | 2 | Emulgator |
| Caprylic/Capric Triglyceride | 5 | Solvent |
| Methylparaben | 0,2 | Konservierungsmittel |
| Propylparaben | 0,15 | Konservierungsmittel |
| Ascorbyl Palmiate | 0,05 | Antioxidationsmittel |
| Aqua | 56,6 | Solvent |
| Propylene Glycol | 5 | Solvent |
| Magnesium Sulfate | 0,8 | Puffersubstanz |
| Parfum | 0,2 | Parfum |

Abbildung 1 zeigt das chromatographische Peakmuster des hergestellten Musters gemäß Beispiel 1:

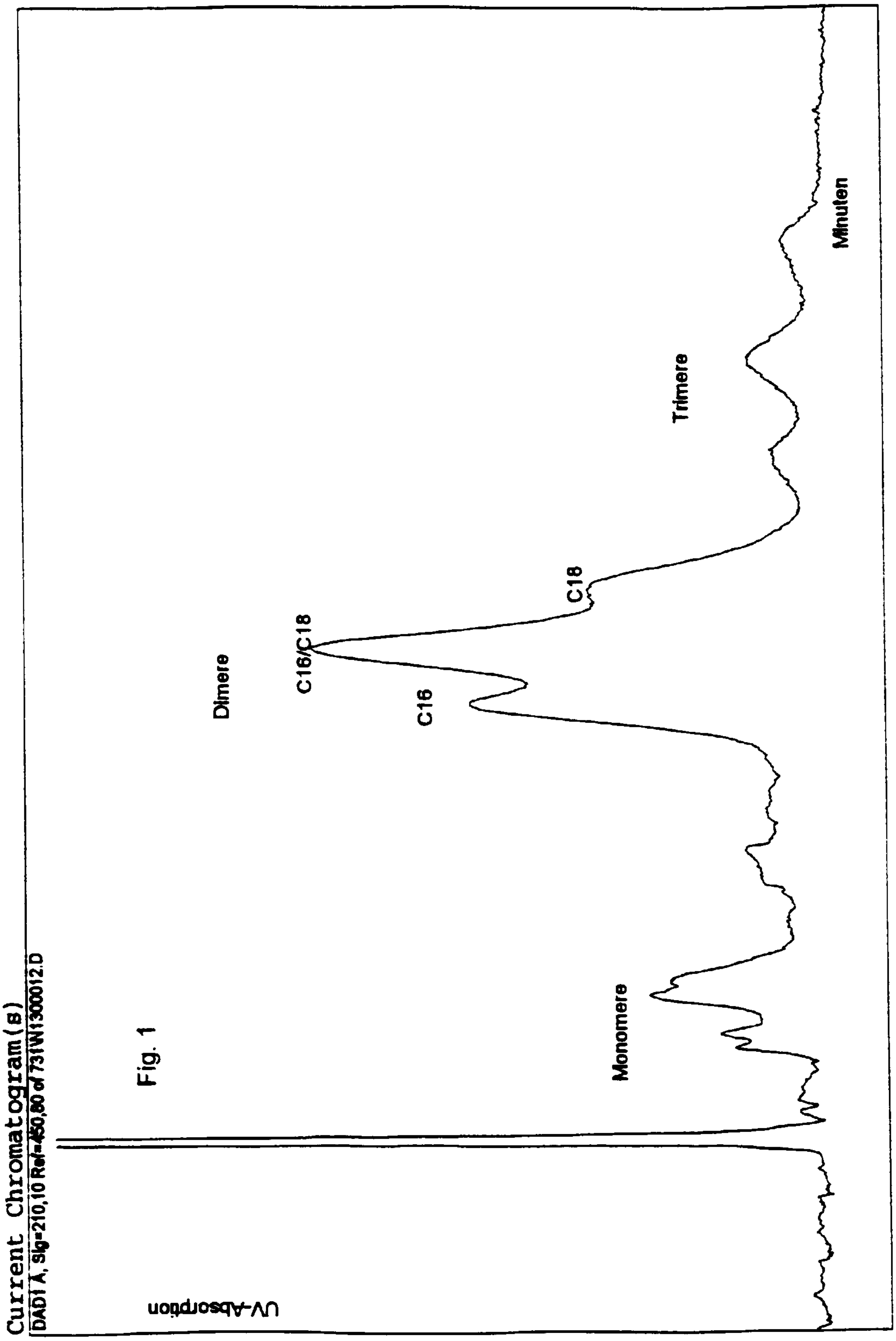
Current Chromatogram(s)
DAD1 A, Sig=210,10 Ref=450,80 of 731W1300012.D
UV-Absorption
Minuten
Trimere
C18
C16/C18
Dimere
C16
Monomere

Fig. 1

**Patentansprüche**

1. Wachsester mit vaselineähnlicher Konsistenz aus einwertigen Alkoholen und Dicarbonsäuren, dadurch gekennzeichnet, daß sie aus mindestens zwei verschiedenen geradkettigen und/oder verzweigten Alkoholen und Dicarbonsäure gebildet sind.

2. Wachsester nach Anspruch 1, dadurch gekennzeichnet, daß sie zu mindestens 80 % durch folgende Struktur gekennzeichnet sind

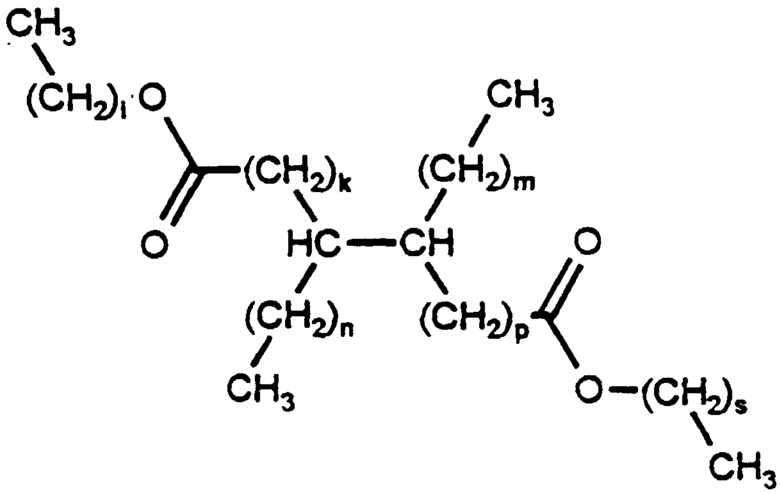

wobei

i einen Zahlenwert von 13 bis 35,
s einen Zahlenwert von 13 bis 35,
die Summe von k+m+n-p etwa 32 beträgt und
$(CH_2)_i$ oder $(CH_2)_s$ - geradkettig oder geradkettig und verzweigt sein kann

3. Wachsester nach Anspruch 1 und 2, dadurch gekennzeichnet, daß als einwertige geradkettige Alkohole solche mit 14 bis 24 C-Atome, vorzugsweise mit 16 oder 18 C-Atomen, eingesetzt werden.

4. Wachsester nach Anspruch 1 und 2, dadurch gekennzeichnet, daß als einwertige verzweigtkettige Alkohole solche mit 14 bis 36 C-Atomen, vorzugsweise Guerbetalkohole, wie 2-Octyldodecanol, oder Isostearylalkohol, eingesetzt werden.

5. Wachsester nach einem oder mehreren der Ansprüche 1 bis 4, dadurch gekennzeichnet, daß als Dicarbonsäure Dimersäuren eingesetzt werden, die durch Dimerisierung von Gemischen aus ungesättigten Fettsäuren hergestellt werden, die mindestens einen C18:1-Anteil von 80 % aufweisen und durch Spaltung von Pflanzenölen mit einem hohen C18:1-Anteil (Ölsäureanteil) gewonnen wurden.

6. Verfahren zur Herstellung von Wachsester mit vaselineähnlicher Konsistenz nach einem oder mehreren der Ansprüche 1 bis 5, dadurch gekennzeichnet, daß Dicarbonsäuren, vorzugsweise Dimersäure, mit zwei verschiedenen geradkettigen und/oder verzweigten einwertigen Alkoholen in Gegenwart eines sauren Katalysators
bei Temperaturen von 100 bis 160 °C
umgesetzt werden, wobei sich ggfs.
eine alkalische Wäsche anschließt.

7. Verwendung der Wachsester mit vaselineähnlicher Konsistenz nach einem oder mehreren der Ansprüche 1 bis 6 als Komponente für Cremes und Salben, vorzugsweise in der Pharmazie, Medizin, Dermatologie und Kosmetik.

Europäisches Patentamt

# EUROPÄISCHER RECHERCHENBERICHT

Nummer der Anmeldung
EP 99 11 2028

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | GB 1 359 327 A (CINCINNATI MILACRON INC) 10. Juli 1974 (1974-07-10)<br>* Seite 7, Zeile 40 - Seite 7, Zeile 42 *<br>* Anspruch 1 * | 1,7 | C08L91/06<br>A61K47/14 |
| X | EP 0 714 929 A (INOUE MTP KK) 5. Juni 1996 (1996-06-05)<br>* Seite 7, Zeile 49 - Seite 8, Zeile 10 *<br>* Seite 9, Zeile 23 *<br>* Beispiel 13; Tabelle 3 * | 1,2 | |
| X | DE 24 36 902 A (UNILEVER EMERY) 20. Februar 1975 (1975-02-20)<br>* Beispiele 1,8-10 *<br>* Ansprüche 1-3 * | 1 | |
| A | WO 94 20067 A (BOOTS CO PLC ;BARTON STEPHEN PETER (GB)) 15. September 1994 (1994-09-15)<br>* Beispiel 3 *<br>* Ansprüche 1,3,5 * | 1-7 | **RECHERCHIERTE SACHGEBIETE (Int.Cl.7)** |
| A | PATENT ABSTRACTS OF JAPAN vol. 006, no. 063 (C-099), 22. April 1982 (1982-04-22) & JP 57 004947 A (TOEI CHEM KK), 11. Januar 1982 (1982-01-11)<br>* Zusammenfassung * | 1-7 | C08L<br>A61K |
| A | DATABASE WPI Section Ch, Week 199022 Derwent Publications Ltd., London, GB; Class E17, AN 1990-168427 XP002121717 & JP 02 110195 A (KAWASAKI STEEL CORP), 23. April 1990 (1990-04-23)<br>* Zusammenfassung * | 1-7 | |

Der vorliegende Recherchenbericht wurde für alle Patentansprüche erstellt

| Recherchenort | Abschlußdatum der Recherche | Prüfer |
|---|---|---|
| MÜNCHEN | 5. November 1999 | Heidenhain, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 99 11 2028

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-11-1999

| Im Recherchenbericht angeführtes Patentdokument | Datum der Veröffentlichung | Mitglied(er) der Patentfamilie | Datum der Veröffentlichung |
|---|---|---|---|
| GB 1359327 A | 10-07-1974 | NL 7114005 A | 16-04-1973 |
| | | CA 946292 A | 30-04-1974 |
| | | DE 2129618 A | 16-12-1971 |
| | | GB 1359969 A | 17-07-1974 |
| | | GB 1359970 A | 17-07-1974 |
| | | US 3961044 A | 01-06-1976 |
| | | US 3981990 A | 21-09-1976 |
| | | US 4045550 A | 30-08-1973 |
| EP 0714929 A | 05-06-1996 | CA 2152846 A | 31-05-1996 |
| | | CN 1123799 A | 05-06-1996 |
| | | JP 8231668 A | 10-09-1996 |
| | | US 5527834 A | 18-06-1996 |
| DE 2436902 A | 20-02-1975 | US 3857865 A | 31-12-1974 |
| | | US 3893931 A | 08-07-1975 |
| | | BE 818393 A | 03-02-1975 |
| | | CA 1047478 A | 30-01-1979 |
| | | CH 606257 A | 31-10-1978 |
| | | ES 428829 A | 16-05-1977 |
| | | FR 2239504 A | 28-02-1975 |
| | | GB 1478373 A | 29-06-1977 |
| | | JP 1262671 C | 25-04-1985 |
| | | JP 50044376 A | 21-04-1975 |
| | | JP 59025835 B | 21-06-1984 |
| | | NL 7410279 A | 04-02-1975 |
| | | US 3912642 A | 14-10-1974 |
| WO 9420067 A | 15-09-1994 | AU 6284494 A | 26-09-1994 |
| | | EP 0789555 A | 20-08-1997 |
| JP 57004947 A | 11-01-1982 | JP 1200524 C | 05-04-1984 |
| | | JP 58034460 B | 27-07-1983 |
| JP 2110195 A | 23-04-1990 | JP 2507562 B | 12-06-1996 |

EPO FORM P0461

Für nähere Einzelheiten zu diesem Anhang : siehe Amtsblatt des Europäischen Patentamts, Nr.12/82